# EUROPEAN PATENT SPECIFICATION

(11) **EP 0 983 086 B1**
(45) Date of publication and mention of the grant of the patent: **29.08.2007**
(21) Application number: 98906248.4
(22) Date of filing: 05.02.1998
(51) Int. Cl.: A61K 39/00, A61K 39/02, A61K 39/12, A61K 45/00, A61K 47/42, A61K 47/44, A61K 9/127, G01N 33/53, G01N 33/543, G01N 33/567

(54) **pH-SENSITIVE LIPOSOMES AND OTHER TYPES OF ENCAPSULATED VACCINES CONTAINING IMMUNOMODULATORS, AND METHODS FOR MAKING AND USING SAME**
PH-SENSITIVE LIPOSOMEN UND ANDERE TYPEN IMMUNOMODULATOREN ENTHALTENDER GEKAPSELTER IMPFSTOFFE SOWIE HERSTELLUNGS- UND ANWENDUNGSVERFAHREN DAFÜR
LIPOSOMES SENSIBLES AU PH ET AUTRES TYPES DE VACCINS ENCAPSULES CONTENANT DES IMMUNOMODULATEURS, ET PROCEDES DE PRODUCTION ET D'UTILISATION

(30) Priority: 05.02.1997 US 37217 P
(43) Date of publication of application: 08.03.2000
(73) Proprietor: Bystryn, Jean-Claude, New York, NY 10016 (US)
(72) Inventor: Bystryn, Jean-Claude, New York, NY 10016 (US)
(74) Representative: Brasnett, Adrian Hugh
(86) International application number: PCT/US1998/002463
(87) International publication number: WO 1998/033520

(56) References cited:
- US-A- 5 182 109
- US-A- 5 422 120
- US-A- 5 585 103
- US-A- 5 591 632
- ADLER A ET AL: "CELLULAR RESPONSES IN PATIENTS TREATED BY LIPOSOMAL ALLOGENETIC MELANOMA VACCINE WITH EITHER SYSTEMIC OR REGIONAL IL-2" PROCEEDINGS OF THE ANNUAL MEETING OF THE AMERICAN ASSOCIATION FOR CANCER RESEARCH. ORLANDO, MAY 19 - 22, 1993, PHILADELPHIA, AACR, US, vol. MEETING 84, 1993, page 493, XP000986100 ISSN: 0197-016X
- BYSTRYN J -C ET AL: "Potentiation of melanoma vaccine activity by IL-2 liposomes" JOURNAL OF INVESTIGATIVE DERMATOLOGY, vol. 106, no. 4, 1996, page 845, XP002296769 & ANNUAL MEETING OF THE SOCIETY FOR INVESTIGATIVE DERMATOLOGY; WASHINGTON, D.C., USA; MAY 1-5, 1996 ISSN: 0022-202X
- BERGERS JOEP J ET AL: "Liposome-based cancer vaccines" JOURNAL OF LIPOSOME RESEARCH, vol. 6, no. 2, 1996, pages 339-355, XP008035500 ISSN: 0898-2104
- CHUN-JUNG CHU ET AL: "PH-SENSITIVE LIPOSOMES" JOURNAL OF LIPOSOME RESEARCH, MARCEL DEKKER, NEW YORK, US, vol. 4, no. 1, 1 March 1994 (1994-03-01), pages 361-395, XP000450216 ISSN: 0898-2104
- J. BIOTECH., January 1996, Vol. 44, JACKSON et al., "Mucosal Immunity: Regulation by Helper T Cells and a Novel Method for Detection", pages 209-216, XP002910830
- FEMS IMMUNOL. MED. MICRO., July 1996, Vol. 14, YOKOYAMA et al., "DNA Immunization: Effects of Vehicle and Route of Administration on the Induction of Protective Antiviral Immunity", pages 221-230, XP002910831
- INFECT. IMMUN., December 1996, Vol. 64, No. 12, MAHON et al., "Interleukin-12 is Produced by Macrophages in Response to Live or Killed Bordetella Pertussis and Enhances the Efficacy of an Acellular Pertussis Vaccine by Promoting Induction of Th1 Cells", pages 5295-5301, XP002910832
- J. EXP. MED., 03 February 1997, Vol. 185, No. 3, MARINARO et al., "Oral But Not Parenteral Interleukin (IL)-12 Redirects T Helper 2 (Th2)-Type Responses to an Oral Vaccine Without Altering Mucosal IgA Responses", pages 415-427, XP002910833
- J. EXP. MED., February 1992, Vol. 175, NAIR et al., "Soluble Proteins Delivered to Dendritic Cells Via pH-Sensitive Liposomes Induce Primary Cytotoxic T Lymphocyte Responses In Vitro", pages 609-612, XP002910834
- CANCER RES., January 1994. Vol. 54, HELLING et al., "GD3 Vaccines for Melanoma: Superior Immunogenicity of Keyhole Limpet Hemocyanin Conjugate Vaccines1", pages 197-203, XP002910835
- ANNALS NY ACAD. SCI., August 1994, Vol. 730, JACKSON et al., "Oral Vaccine Models: Multiple Delivery Systems Employing Tetanus ToxoidAlpha ", pages 217-234, XP002910836
- J. VIROL., July 1993, Vol. 67, No. 7, NAIR et al., "Induction of Primary, Antiviral Cytotoxic and Proliferative Responses with Antigens Administered Via Dendritic Cells", pages 4062-4069, XP002910837
- Oratz, R. et al (1996) Proc. Am. Assoc. Clin. Oncology 15:436
- Gershman, N. et al (1994) Vaccine Research 3:83-92
- Collins, D.S. et al (1992) J. Immunol. 148:3336-3341

## Description

This application claims priority, from U.S. Provisional Application No. 60/037,217, filed February 5, 1997.

### BACKGROUND

Many vaccines do not provide as full and effective an immune response as is desired. This is particularly true of cancer vaccines and vaccines for some infectious diseases. One reason is failure to stimulate CD8+ T cells, another reason is that the immune responses which are induced are often weak.

CD8+ cells are cytolytic T lymphocytes (also known as CTLs) and are capable of destroying other cells. They are the principal mediators of protective immunity both against cancer and against certain intracellular pathogens. For that reason it is important that vaccines be able to stimulate CD8+ or cytolytic T cells that react specifically to the antigen(s) in the vaccine in order to maximize the clinical effectiveness of the vaccines.

However, exogenous antigens. i.e. antigens introduced from outside the body such as those in vaccines, are unable to provoke an effective CD8+ T cell response because exogenous antigens are not processed and presented by the route that is necessary to stimulate these cells (though such antigens do stimulate other types of immune responses).

Exogenous antigens are taken up by the immune system's antigen presenting cells and are ultimately displayed on these cells' surfaces, where they are "seen" by immune cells that thereby learn to react against these antigens. However, exogenous antigens such as those in vaccines are processed by a pathway that leads to their presentation in conjunction with class 11 histocompatibility (HLA) cell-surface molecules. CD8+ T cells do not respond to antigens that are presented with class II molecules. CD8+ T cells do respond to antigens presented by a different type of HLA molecules, the class I HLA molecules, which are also present on the surface of antigen presenting cells. However, only antigens which are synthesized by the cells themselves, and released into the cytosol (cell's interior), enter the pathway that results in presentation by class I HLA molecules (Bevan, Nature 325; 192, 1987; Davis and Bjorlunan, Nature 334;395, 1988; Moore et al, Cell 54:777, 1988).

However, by using specialized carriers or molecules it is possible to trick the body into mounting a CD8+ or cytolytic T cell response to exogenous antigens. These carriers cause exogenous antigens to enter the cytosol and to be presented through the class I pathway of antigen-processing (Moore et al, Cell 54:777, 1988; Reddy et al, J Immunol Methods 141:157, 1991; Nair et al, J Immunol Methods 152:237, 1992), so that they can now stimulate CD8+ T cell responses. These specialized carriers include liposomes (pH-sensitive or lipophilic polylysine [Nair et al, J. Immunol Methods 152:237, 1992]; virosomes [Manning and Gould-Fogerite, Biotechniques 6:682, 1988]; particulate beads or particles made of glass, iron, biodegradable or other material); heat shock proteins; certain toxins; and certain other molecules.

One particular means which is known to raise a CD8+ T cell or cytolytic T cell response to a given antigen is to encapsulate the antigen into pH-sensitive liposomes (Reddy et al, J. Immunol Methods 141:157, 1991; Straubinger, Method Enzymol 221:301, 1993; Martin et al, Immunology Letters 37:97,1993; Collings et al, J Immunol 148:3336,1992), which are specialized liposomes that fuse with cell membranes at an acidic pH, and are safe for use in humans (Patel, In: Liposomes as Drug Carriers: Recent Trends in Progress ed. Gregoriadis, (John Wiley, Chichester, 1988) pp.51-62; Tan and Gregoriadis, Biochem Soc. Transactions 17:693,1989; Abraham and Shah, J Immunol 149:3719, 1992). When a pH-sensitive liposome is taken up into an antigen-presenting cell, the acidic pH of endocytic vacuoles protonates the pH-sensitive lipid of the liposome, which then fuses with the membrane of the vacuole (Reddy et al, J Immunol Methods 141:157.1991; Bllens et al, Biochemistry 23:1532,1984). The contents of the liposome (e.g. the encapsulated antigen[s]) is then released-into the cytosol, can enter into the class I pathway of antigen processing, and can be presented by HLA class I molecules and stimulate CD8+ T cell responses. In contrast, conventional (non pH-sensitive) liposomes are not able to accomplish this (Moore et al, Cell 54:777. 1988; Nair et al, J Exp Med 175:609,1992; Harding et al, J Immunol 147:2850,1991; Straubinger et al, FEBS Lett 179:148, 1985; Chu et al, Pharm Res 7:824,1990) and are not effective carriers to stimulate CD8+ or cytolytic T cell responses. Other types of carriers and molecules can deliver exogenous antigen(s) into the endocytic pathway of antigen processing and presentation by other mechanisms as noted above.

Even though it is possible to stimulate CD8+ T cells with a specialized carrier such as a pH-sensitive ilposome, many antigen or vaccine induced immune responses remain too weak to be effective. Thus, it is not only important to induce a CD8+ T cell response, but also to find a way to enhance the magnitude of CD8+ T cell responses and other types of immune responses which play a role in mediating protective immunity. One way to do so is to administer one or several immunomodulators together with the vaccine.
Immunomodulators are specialized proteins that enhance and amplify immune responses Induced by antigens. Such immunomodulators include cytokines such as IL-1, IL-2, IL-6, IL-12, GM-CSF, and others that remain to be discovered, Interleukin-2 (IL-2) and granulocyte-macrophage colony-stimulating factor (GM-CSF) are particularly interesting for their roles in stimulating T cells and antigen presenting cells, respectively. IL-2 stimulates the development of T cells which have been activated by an antigen, and help to generate clones of T cells specific for a given antigen. GM-CSF activates and stimulates antigen presenting cells (Taglietti in Dendritic Cells in Fundamental and Clinical Immunology, Vol-2, ed. Banchereati and Schmitt (Plenum Press, New York, 1995) pp.565-569; Fischer et al. Bur. J. Immunol. 18:1151, 1988), effectively making antigens more immunogenic. However, these immunomodulators cannot stimulate a CD8+ or other type of T cell response to antigens that otherwise would not cause such a response. They only magnify the strength of the response(s) which is induced.

Immunomodulators have previously been used in vaccines in conjunction with conventional liposomes. This use has nothing to do with inducing a CD8+ T cell response, but is intended to overcome the drawback that immunomodulators have a short circulatory half-life. It has been found that conventional liposomes can act as slow release vehicles to prolong the half-fife of the encapsulated immunomodulators at the site of injection and in the circulation, and avoid high and toxic dosage levels that otherwise would be required to maintain the same blood levels (Patel, In: Liposomes as Drug Carriers: "Recent Trends in Progress" ed. Gregoriadis. (John Wiley, Chichester, 1988) pp.51-62: Tan and Gregoriadis, Biochem Soc Transactions 17;693, 1989; Abraham and Shah, J Immunol 149:3719,1992; Mbawuike et al, Vaccine 8:347, 1990; Talmadge et al, Cancer Res 47:5725,1987; Gershman et al, Vaccine Res 3(2):83-92, 1994).

Encapsulation of a vaccine and an immunomodulator in a slow-release vehicle such as a conventional liposome does not result in a CD8+ T cell response, but in an entirely different response, a CD4+ response, mediated by a different class of T cells which react to antigen bound to class II HLA molecules, not to class I HLA molecules.

Obtaining a strong CD8+ T cell or CTL response to an antigen is especially important in the area of cancer vaccines and vaccines for some infectious diseases such as AIDS. Melanoma is an example of an important target for vaccine treatment, due to its increasing prevalence and poor prognosis, and to the fact that melanoma appears amenable to vaccine treatment.

Vaccines are a promising treatment for melanoma. They prevent melanoma in animals and slow its progression in humans. The beneficial effects of vaccine treatment are mediated by stimulating antitumor immune responses. Unfortunately, melanoma vaccines are poorly immunogenic. A major challenge in the construction of an effective vaccine for melanoma is to develop procedures that boost vaccine immunogenicity, especially their ability to stimulate a strong CD8+ T cell or CTL response (American Cancer Society: Cancer statistics 1990. CA 40:9, 1990; Bystryn et al. in Biologic Therapy of Cancer, 2nd Edition (ed. DeVita et al.) J.B. Lippincott, Philadelphia, PA, 1995, pp.668-679; Bystryn, I. Immunol. 120:96, 1978; Hersey et al. Cancer Immunol. Immunother. 25:257, 1987; Livingston et al. PNAS(USA) 84:2911,1987; Mitchell et al, Cancer Res 48:5883, 1988).

Bystryn et al (1996) J. Invest. Dermatology 106:845 discloses that an IL-2 liposome provides a safe and potent method of augmenting the immunogenicity of melanoma vaccine.

Adler et al (1993) Proc Am Assoc Cancer Research 84:493 assesses the biological effects of allogeneic liposome embedded human melanoma vaccine combined with IL-2 and cimetidine in metastatic melanoma patients.

Oratz et al (1996) Proc Am Assoc Clin Oncology 15:436 reports that IL-2 liposomes are safe, markedly potentiate the immunogenicity of a melanoma vaccine and may induce meaningful clinical responses.

Until this invention, it was possible to gain a strong response to an antigen by pairing a vaccine and an immunomodulator, and it was possible to obtain a CD8+ T cell or CTL response to an antigen by encapsulating it into pH-sensitive liposomes or other specialized carriers. However, the problem of inducing an immune response that is the desirable CT8+ T cell or CTL response and at the same time stimulate that response to an effective strength had not been solved.

Until this invention no one has attempted to solve the problem of concurrently inducing and potentiating a particular and desirable immune response (the CD8+ T cell or CTL response) by using a pH-sensitive liposome or other specialized carrier to permit the antigen to be presented by HLA class I molecules and to concurrently administer an immunomodulator with the antigen so that the immunomodulator is present at the site where it is needed to exert its effect.

### SUMMARY OF THE INVENTION

The present invention provides a vaccine composition which comprises: at least one cancer antigen prepared from shed antigens of a cancer cell; at least one immunomodulator selected from IL-1, IL-2, IL-4, IL-6, IL-12 and GM-CSF; and a carrier which is a chloroform-free pH sensitive liposome comprising dioleoylphosphatidylethanolamine (DOPE) and cholesteryl hemisuccinate (CHEMS) which are formed at a pH of 8,5-9.5, to encapsulate said cancer antigen and said immunomodulator and to deliver them together to immune cells when administered to a human, such that said antigen induces a CD8+ T cell response.

In one aspect, the liposomes are formed at the highest concentration of lipid possible, to maximise the amount of antigens and immunomodulators that can be encapsulated.

In another aspect, the mixture of antigen(s), immunomodulator(s) and carrier are used without removing from the mixture the antigen(s) and immunomodulator(s) which have not been bound or incorporated into the carrier.

The vaccine composition of the invention may be used in a method of treatment of the human or animal body. The invention also provides the use of said vaccine composition in the manufacture of a medicament to induce an immune response against a cancer and so treat or prevent the cancer. A particular application of this invention is in the area of melanoma vaccines, and an improved treatment for the primary and secondary prevention of this cancer.

A special object of this invention is a vaccine in the form of a polyvalent vaccine for melanoma which includes multiple melanoma antigens encapsulated into pH-sensitive liposomes with one or all of IL-2, IL-12 and GM-CSF which is especially desirable for obtaining a strong CD8+ T cell or CTL response against melanoma.

The compositions of the present invention contain cancer antigens shed or released into culture medium by cancer cells that express and shed or release cancer associated antigens.

The immunomodulators used in the present invention increase the magnitude and frequency of the immune responses induced by the antigens, of the vaccines of the present invention.

The present invention provides an improved use of vaccines as the vaccine of the present invention provides, together, the carrier which permits CD8+ T cell responses to be induced, an immunomodulator that magnifies these responses and an antigen that stimulates these responses together, rather than administered separately, as had been provided prior to the present invention. The composition of the present invention allows administration of lower doses of the individual components to have a greater effect.

Many vaccine induced immune responses have been found to be weak and hence ineffective. This is particularly true for cancer and infectious disease vaccines which are based on pure proteins or peptides. One method to increase the immune response to these vaccines has been to use immunomodulators as vaccine adjuvants to facilitate or amplify antigen driven immune responses. Examples of immunomodulators include 1L-1, IL-4, IL-2, IL-6, IL-12 and GM-CSF. Two important (imitations to using immunomodulators as vaccine adjuvants are 1) their short half-life in the circulation or site of injection (IL-2, for example, has a half-life of less than half-an-hour) and 2) the normal practice of administering the immunomodulator separate from the vaccine.

Administering the immunomodulator in a carrier containing the antigen both prolongs its half-life and delivers it in close proximity to the vaccine or antigen. These carriers are taken up by the lymphatic system after subcutaneous injection and thus delivers incorporated antigens and immunomodulators directly to draining lymph nodes where they are needed to stimulate immune responses. Moreover, liposomes, for example, have been demonstrated to be a slow release vehicle for IL-2, for example. Following subcutaneous injection in animals, 50% of liposome encapsulated IL-2 remained at the injection site for 24 hours, as compared to less than 2% for free IL-2, and biologically active IL-2 was present in the circulation for over 72 hours while none was present after 6 hours if given in free form. Anderson PM, Katsanis E, Spencer SF, Hasz D, Ochoa AC. Bostrom B. Depot characteristics and biodistribution of interleukin-2 liposomes: Importance of route of administration. J Immunother 12:19-36 (1992) Preclinical studies have also shown that antibody and cellular responses to bacterial and viral vaccines and cellular and protective immune responses to cancer vaccines incorporated into conventional liposomes containing IL-2 are greater and longer lasting than if the vaccines are administered with free IL-2. Tan L, Gregoriadis G, Effect of interleukin-2 on the immunoadjuvant action of liposomes, Biochem Soc Transactions 17:693-694 (1989); Mbawuike IN, Wyde PR, Anderson PA, Enhancement of the protective efficacy of inactivated influenza A virus in aged mice by IL-2 liposomes, Vaccine 8:347-352 (1990); Anderson P, Katsanis E, Leonard A, Show D, Loeffler C, Goldstein M, Ochoa A, Increased local antitumor effects of interleukin 2 liposomes in mice with MCA-106 sarcoma pulmonary metastases, Cancer Res, 50:1853-1856 (1990); Abraham E, Shah S, Intranasal immunization with liposomes containing IL-2 enhances bacterial polysaccharide antigen-specific pulmonary secretory antibody response, J Immunol, 149:3719-26 (1992). Melanoma vaccines, for example, encapsulated into IL-2 conventional (not pH-sensitive) liposomes stimulated antimelanoma cytolytic antibodies, delayed type hypersensitivity, and tumor protective immune responses more effectively than vaccine encapsulated into liposomes lacking IL-2 or given with free IL-2. Gershman N, Johnston D, Bystryn J-C, Potentiation of B16 melanoma vaccine immunogenicity by IL-2 liposomes, Vaccine Res 3(2):83-92 (1994). While these studies indicate that encapsulating immunomodulators into conventional liposomes enhances the adjuvant activity of the immunomodulator and of the liposomes, substitution of conventional liposomes with specialized or pH-sensitive liposomes or other vehicles which permit stimulation of CD8+ T cell has provided a previously unknown and unexpected benefit in improving the immunogenicity of the vaccines.

Antigens encapsulated into conventional liposomes are processed very differently from antigens encapsulated into pH-sensitive liposomes. The antigens in conventional liposomes are presented in conjunction with class II histocompatibility molecules and as a result stimulate CD4+ T cells. In contrast, antigens in pH-sensitive liposomes can be presented in conjunction with class I histocompatibility molecules, stimulating a different class of cells, the CD8+ T cells. The effect of such differences on the activity of an immunomodulator is unknown. Therefore results of encapsulating an antigen(s) together with an immunomodulator into a pH-sensitive liposome or another carrier or molecule that allows the antigen(s) to enter the endocytic pathway of antigen processing and presentation is difficult to predict.

The present invention employs an improved pH sensitive liposome which has been prepared from dioleoylphosphatidylethanolamine (DOPE) and cholesteryl hemisuccinate (CHEMS), preferably as the sole lipasome-forming lipids; which are made by dissolving the lipids in methanol, in the absence of chloroform, at a preferred ratio of 6:4; and at a preferred pH of about 8.5 to about 9.5, and which are constructed from an amount of lipids in the range of 8 to 80 mg of lipid per 0.4 ml dose of vaccine.

The present invention-also provides an improved method of using pH-sensitive liposomes. The improvement is to use them at a high concentration of lipid per dose of vaccine as this results in greater incorporation of antigen or vaccine into the liposome and in increased ability to simulate cellular immune responses in general and CD8 + T cell responses in particular.

In another embodiment of the present invention, the composition of the invention may contain additional ingredients, such as pharmaceutically or therapeutically acceptable carriers or diluents and additional adjuvants, unencapsulate or unbound immunomodulators, antigen or carrier.

Preferred antigens of the present invention include antigens shed by melanoma cells in culture.

The present invention also relates to methods of preventing melanoma; cancers, for example of the breast, lung, colon, prostate, stomach, gastrointestinal tract, brain, blood cells; which method includes administering to a mammal, such as human, a preventive effective amount of the vaccine of the present invention wherein the antigen of the vaccine induces a CD8+ T cell immune response. The present invention also relates to a method of treating these diseases wherein the vaccine of the invention is administered to a mammal, such as a human, in an amount sufficient to eliminate or reduce the severity of the disease or treat the disease. In a further embodiment, the present invention relates to a method of inducing the CD8+ T cell response or increasing the CD8+ T cell response to an antigen. A method of making such compositions is also part of this invention.

An improved assay for detecting and quantifying antigen-specific CD8+ T cells is also part of this invention. This assay is a major modification of a previously described ELISPOT assay. King, C.L., Thyrbronitis, G., Nutman, T.B., J. Immunolog Methods, 132:37-43 (1970) It can be used to measure the ability of vaccines, such as vaccines constructed as described in this invention, to stimulate antigen-specific CD8+ T cell responses. The assay quantitates in a blood or other sample the number of CD8+ T cells that respond to an antigen of interest, as for example an antigen which is provided to a patient in a composition of this invention. The advantages of this assay is that it is a direct assay that does not require, and avoids the artifacts of, prolonged in vitro stimulation with antigen which is required with currently available assays such as in vitro stimulation and limiting dilution analysis assays. In addition this new assay is more sensitive and rapid than traditional cytolytic or cytokine release assays; it measures the actual number of CD8+ T cells rather than precursor cells; does not require that the CD8+ T cells be purified or cloned prior to assay; and is sensitive enough to quantitate in periphereal blood the small number of antigen specific CD8+ T cells induced by vaccine immunization. It can be used to measure responses to whole cells or to individual antigens such as peptides.

The assay is based on measuring the number of CD8+ T cells that have been stimulated by a target antigen as evidenced by their secretion of certain cytokines. In a preferred aspect of this method, the cytokine(s) secreted by antigen stimulated CD8+ T cells is captured and immobilized by conducting the assay in a vessel that contains a membrane coated with antibody directed against the cytokine whose secretion is being measured, and then visualizing the captured cytokine by means known in the art of immunoassays. The preferred method of the present invention includes providing a container that contains a membrane with anti-cytokine coated antibody to which is added a test sample which contains monocyte-depleted, effector cells, such as human peripheral blood lymphocyte which are mixed with target cells that present a target antigen of interest bound to Class I HLA molecules, and antibodies which block Class II HLA molecules. Cytokine secreting, antigen-activated, CD8+ T cells are then detected by adding another cytokine antibody which has been labeled in such a fashion as to be visualized as with an ELISA procedure. In a preferred embodiment of this method, the first anti-cytokine antibody is bound to a PDVF membrane on the bottom surface of the container. The measurement or detection of cytokine released by activated CD8+ T cells as well as blocking of Class II HLA molecules and monocyte depletion of the patient lymphocyte-enriched sample ensures the effector cells measured or detected are CD8+ T cells. One of ordinary skill will appreciate that appropriate controls are needed to ensure validity of any measurement. Such controls include the ability to block the reaction with antibody to CD8+ T cells but not to CD4+ T cells or other types of lymphocytes, the ability to block the reaction with antibody to class I HLA but not with anti-HLA class II antibody; and testing test samples to target cells that present an antigen other than the test antigen.

Several critical modifications of the previously described ELISPOT assay have been introduced; to make the assay more sensitive and reliable. These include conducting the assay with a large number (approximately a 10 fold greater number) i.e., 500,000 cells/test sample instead of the usual number of 50,000 cells/test sample of effector lymphocytes so as to detect the very small number of vaccine induced CD8+ T cells which are present within the effector cell population. Another modification is to conduct the assay in the presence of antibodies to class II HLA molecules, so as to prevent certain populations of cells unrelated to CD8+ T cells of being mistakenly counted as CD8+ T cells. Other modifications includes depleting monocytes from the effector lymphocyte population to reduce background interference with the results of the assay and to measure more specifically the number of cells which are CD8+ T cells; performing the assay in the continued presence of test peptide rather than removing test peptide that is not bound to target cells prior to assay, pre-treating target cells with interferon for 2 days prior to use to increase their expression of class I HLA and hence the amount of test peptide they can present, and preparing the blocking antibodies that are used in the assay by concentrating them by dialysis to minimize denaturation and to remove contaminating impurities hence improves their activity.

The assay is performed by isolating lymphocytes from a sample of blood obtained from a subject whose immune status is of interest, for example a subject immunized to a target antigen(s) or having a condition such as cancer or an infection to which an immune response may occur. Isolating lymphocytes from whole blood with Ficoll-Hypaque is a well-known technique. The sample may be fresh or reconstituted, for example from a frozen or otherwise preserved sample. The number of lymphocytes in the sample must be sufficiently large to detect the very small number in periphereal blood which is induced by vaccine treatment. That number should be in the range of 500,000 or more lymphocytes/well. The lymphocytes include various types of immune cells including CD8+ T cells; but are artificially depleted of monocytes, which have activity that would prevent identification of CD8+ T cells by antibody-blocking later in the assay. The lymphocytes (effector cells) are incubated on an appropriate surface such as a membrane or plate that can capture the cytokine(s) they release once activated by antigen. A preferred membrane is PDVF membrane, and a convenient type of plate is a multiwell microtiter plate. The surface is precoated with anticytokine antibodies so as to trap cytokines secreted by individual CD8+ T cells that have been stimulated by antigen, and by this means to visualize the activated CD8+ T cells. The cytokine that is measured can be any cytokine produced and secreted by an antigen activated CD8+ T cell. Interferon, in particular interferon-gamma, is a preferred cytokine. The effector cells are incubated with cells that present the target antigen on their surfaces.

The target cells may be tumor cells or infectious organisms which already bear a target antigen, or antigen presenting cells which have been incubated with a selected target antigen such as an isolated peptide under conditions such that the target antigen is transferred to the cell's surface. In order to provide the most effective assay, the cells presenting target antigen should present the antigen in conjunction with class 1 HLA molecules - which are the type of HLA molecules to which CD8+ T cells respond. If the desired target cells do not have class I HLA molecules. DNA encoding such molecules may be inserted into the cells by known methods to cause the cells to express class I HLA molecules. A preferred class I HLA molecule is the known allele HLA-A2. To additionally insure that the responding cells in the assay are CD8+ T cells that react to antigen presented in the context of class I HLA molecules, antibodies to HLA class II antigens are added to the jointly incubated effector and target cells in order to prevent any target antigen from being presented in conjunction with HLA class II molecules by blocking the molecules with the antibodies. Finally, the type of cells stimulated and their HLA restriction is confirmed by adding antibodies to antigens which appear on CD8+ and CD4+ T cells and to class I and II HLA molecules to a certain number of wells, and determining that the CD8+ T cell response has been blocked by antibodies to CD8 and class I HLA, but not by antibodies to CD4 and class II HLA. In order to provide the most effective assay, the target cells can be pre-incubated with interferon-gamma for 2 days prior to use to increase the amount of class I HLA molecules present on their surface and hence the amount of test antigen or peptide that can be presented. In order to provide the most effective assay, test peptide or antigen should not be washed off or otherwise removed from target cells prior to incubating effector cells with target cells.

After the samples have been incubated for a suitable time and under suitable conditions for any CD8+ T cell to have been stimulated by the target antigen and to secrete cytokine in response, the cells which have secreted cytokine are visualized and counted by any conventional means, for example by using a double sandwich ELISA (see Taguchi et al 1990). Each "dot" appearing on ELISA corresponds specifically to a single cell which has secreted cytokine in response to stimulation. A positive CD8+ T cell response is seen by obtaining cells which are determined to be CD8+ T cells by virtue of having the CD8 antigen and by having reacted to class I HLA molecules on the antigen presenting cells. That the CD8+ T cells are specific for the target antigen is seen by their lack of reaction to target cells containing control antigens that can be recognized by CD8 and have the same HLA restriction as the target antigen, but are unrelated to the target antigen. That these CD8+ T cells are activated is also seen by their secretion of the cytokine. The assay is very sensitive and is capable of detecting 0.001 % of CD8+ T cells present in the effector cell preparation. Five or more CD8+ T cells out of 500,000 effector cells may be considered a positive result in this assay. The general techniques and materials used to carry out the assay are described subsequently.

Accordingly, disclosed herein is an assay for determining whether a CD8+ T cell response to a target antigen has occurred in a subject by performing the steps of coating a PDVF (polyvinylidene fluoride) membrane surface with anticytokine antibodies, adding to the coated membrane target cells which carry the target antigen on their surfaces, obtaining a sample of peripheral blood cells from the subject and depleting the sample of monocytes to provide effector cells, adding the effector cells to the cell suspension to obtain test samples, adding to the test samples antibodies which block class II HLA molecules, adding to some of the test samples antibodies which bind to CD8+ T cell surface antigens, incubating the resulting test samples for a time sufficient to allow any CD8+ T cells in the test samples which recognize the antigen to respond to the antigen by secreting cytokine, and measuring the total number of cells which recognize the antigen by counting the number of cells which react with anticytokine antibodies, and determining the number of cytokine secreting cells which are CD8+ by subtracting the number of reactive cells in wells containing anti-CD8 antibodies from those in wells containing a control antibody. An essential element of the assay if that the target cells and the cells in the test sample share the HLA allele to which the test antigen can bind to.

### BRIEF DESCRIPTION OF THE DRAWINGS

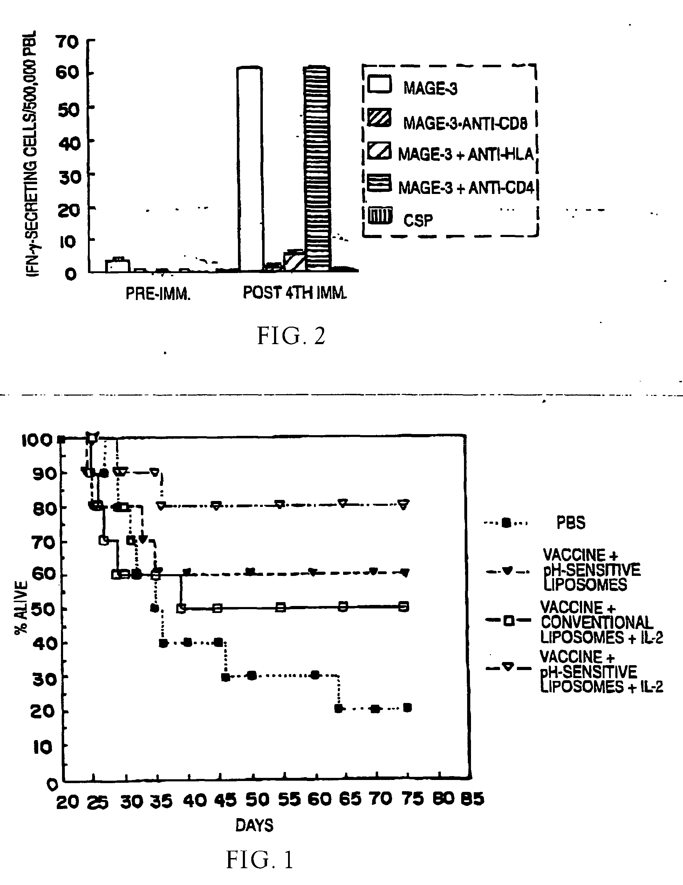

Figure 1: Improved survival of mice immunized to vaccine encapsulated into pH-sensitive liposomes containing IL-2. Groups of 10 mice were immunized weekly x 4 with melanoma vaccine encapsulated into pH-sensitive liposomes (▼), vaccine encapsulated together with 10,000 international units of IL-2 into pH-sensitive liposomes (∇), vaccine encapsulated together with IL-2 into conventional (non-pH-sensitive) liposomes (□), or PBS (■). Two weeks after the last immunization, all mice were challenged with a lethal number of melanoma cells. Note that the best survival was that of mice immunized to vaccine encapsulated together with IL-2 into pH-sensitive liposomes.
Figure 2: Filter spot assay for vaccine-induced CD8+ T cells to the melanoma antigen MAGE-3. Monocyte depleted PBL from an HLA-A2 positive patient obtained before (pre-) and after four melanoma vaccine immunizations were assayed for reactivity to an HLA-A2 positive target cell line that had been pulsed with A2.1 restricted peptides derived from MAGE-3 or CSP (an A2.1 restricted control peptide). Note that there are an increased number of cells responding to MAGE-3 peptide pulsed cells after vaccine immunization. These cells are CD8+ (as the reactivity is abrogated by anti-CD8); HLA-restricted (as reactivity is completely abrogated by anti-HLA class 1 antibody); and is peptide-specific (as there is no increase in reactivity to CSP-peptide pulsed cells).

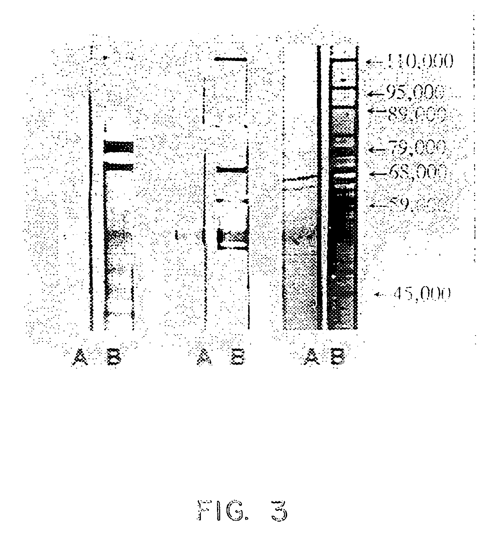

Figure 3: Vaccine-induced antibody response to individual antigens expressed in vivo.
The figure illustrates the reaction of antibodies in pre-(lane A) and post-(lane B) vaccine treatment sera in 3 patients. The results were obtained by immunoblotting using fresh, surgically resected melanoma tissue as the antigen source. Note that vaccine immunization induced antibody responses to one or more antigens of 45, 59, 68, 79, 89, 95 and/or 110 kD

### DETAILED DESCRIPTION OF THE INVENTION

This invention is directed to compositions that increase the immunological activity of antigens and vaccines. Such a composition includes a shed antigen of a cancer cell to which an immune response is desired, a pH-sensitive liposome as defined herein above to permit the antigen to stimulate a CD8+ T cell or CTL response, and one or more immunomodulators as defined herein above to augment the magnitude of the immune responses which are stimulated. The antigen and immunomodulator may be encapsulated together or otherwise bound in or with the pH-sensitive liposome or other carrier, so that the antigen(s), immunamodulator(s), and carrier are delivered together to immune cells.

In particular, a composition of this invention is designed to stimulate a particular and effective immune response, the CD8+ T cell or CTL responses which mediate the killing of undesired target cells. When such a response is desired, each element of the composition may have a role in eliciting the response at the desired level of effectiveness. The composition includes the antigen(s) of choice, to which one wishes to elicit the immune response, the one or more immunomodulator, which is expected to enhance the level of immune responsiveness; and the carrier, which introduces the antigen into the class I restricted pathway of antigen processing, and presentation so that the antigen can be presented to CD8+ cells or CTLs in conjunction with class I histocompatibility antigens on the cell surface, so as to elicit CD8+ or CTL, responses. The carrier also acts to deliver the antigen(s) and the immunomodulator(s) together to immune cells, where both are needed in close proximity to each other to exercise their function, and may also act as a slow release vehicle to prolong the duration of effect of the antigen(s) and immunomodulator(s).

An antigen is any entity that stimulates an immune response either independently or together with a carrier or adjuvant. Thus the antigen of the composition may be any shed antigen of a carrier cell to which an immune response is desired. Antigen as used herein may signify a single antigen or more than one antigen, or a vaccine. Any antigen may contain more than one antigenic determinant to which a specific immune response occurs.

As is well known, antigens may be obtained from many sources, natural and synthetic. For example, natural antigens may be The antigen may be a fragment of the surface, or an internal fragment, derived from any of this source. Such antigens are likely to be proteins and peptides, polysaccharides, gangliosides or nucleic acids. Antigens may also be artificially produced by well-known methods of chemical synthesis, protein synthesis, or genetic engineering. Methods for obtaining antigenic materials and purifying antigens are well known in the art, as are methods for synthesizing a desired antigen. In addition, assays for determining that a given entity is immunogenic and will function as an antigen are well known, and an improved assay to measure one such response, the CD8+ T cell response, is part of this invention..

The compositions of this invention may include a single antigen or multiple antigens. Multiple antigens may be directed to the same condition so as to strengthen the immune response to that condition, or may be directed to different conditions to provide an immune response to several conditions. A vaccine signifies one or a collection of multiple antigens, and may also include a medium, a preservative, an adjuvant, and other known vaccine components including pharmaceutically acceptable carriers or additives. A polyvalent vaccine may contain antigens from the same source and elicit an immune response to a single disease or condition or may contain antigens from different sources and elicit an immune response to more than one disease or condition. For example, using a polyvalent vaccine containing a broad range of tumor antigens increases the chance that the vaccine contains antigens able to stimulate protective immunity. Furthermore, immune responses to multiple antigens are more likely to result in killing of a tumor cell than a response to a single antigen. Also, a broad range of antigens is more likely to circumvent antigenic heterogeneity.

The ability to stimulate enhanced immune responses, in particular a CD8+ T cell or CTL response, against cancer cells so as to enable the immune system to contain and destroy such cells, is a particular object of this invention. The antigen concerned may be a whole cell of any type that has been transformed and as a result has the characteristics of a cancer or diseased cell. Or the antigen may be a cell surface or internal fragment of such a cell shed by such a cell into bodily fluid or culture medium. Such an antigen may be a protein or a polysaccharide, or a ganglioside or a nucleic acid, or any other entity capable of eliciting an immune response against cancer. As noted above, antigens may be naturally or synthetically produced by known methods, for example by culturing cancer cells and isolating desired antigens therefrom, or by isolating desired antigens from cancer tissue or by synthesis or by genetic engineering.

The preferred immunomodulators are IL-2, L-12, IL-1 and GM-CSF. IL-2 is produced by helper T cells and is known to cause antigen-activated T cells to undergo clonal expansion (i.e. divide to form more T cells also reactive to the antigen). IL-12 enhances both antigen-specific CD8 + CTL responses and complement-fixing antibody production *in vivo.* Mice immunized with IL-12 plus synthetic HIV peptide in adjuvant show a significant increase in peptide-specific CTL induction. Ahlers, J.D., et al., ''Cytokine-in-adjuvant steering of the immune response phenotype to HIV-1 vaccine constructs," J. Immunol., 158:3947-3958 (1997). Similarly, IL-12 plus protein antigens increase antigen-specific IgG2a, IgG2b, and IgG3 antibody production by 10-1000-fold. Germann, T., et al., "Interleukin-12 profoundly up-regulates the synthesis of antigen-specific complement-fixing IgG2a, IgG2b and IgG3 antibody subclasses in vivo," Eur. J. Immunol., 25:823-829 (1995). These effects, along with the ability of IL-12 to shift T-cell populations towards a Th1 phenotype (Zeh, III. H.J., et al., "Interleukin-12 In: The Cytokine Handbook. 2nd Edition," ed. by A. Thomson; Academic Press, New York, NY, pp. 239-256 (1994)), increase the likelihood of an effective cellular immune response. GM-CSF is produced by various types of cells and stimulates antigen-presenting cells. These proteins are commercially available (for example from Chiron [Emeryville, CA] and lmmunex [Seattle, WA] respectively) or may be isolated from the cells which produce them by known methods.

The carrier of this invention is any entity which is capable of causing the antigen to be presented on the cell surface of an antigen presenting cell in conjunction with class I histocompatibility molecules so as to stimulate CD8+ T cells or CTL and other immune cells. As noted above, several entities are known to have this effect, among them specialized liposomes and virosomes, molecules such as heat shock proteins and toxins, and small particulate objects such as glass, iron or biodegradable beads. Obtaining any such entities and using them in a composition of this invention will be readily accomplished by a skilled person. A preferred carrier is a special type of liposome - the pH-sensitive liposome. These liposomes fuse with cell membranes or endosomes to release their contents into the interior of the cell. Preparation of pH-sensitive liposomes is known in the art and an improved method for preparing and using such liposomes is provided in the Methods section.

The compositions of this invention are made by combining one or more antigens to which an immune response is desired, one or more immunomodulators, and one or more carrier capable of delivering the antigen such that the antigen is presented on the cell surface in conjunction with class I histocompatibility molecules. The amounts of each component are those amounts effective to induce an immune response, preferably a CD8+ T cell or CTL response, to the antigen. That such responses have been induced may be determined by use of assays well known in the art. This invention also provides a new and superior assay that may be used to determine that a composition of this invention to induces a CD8+ T cell response. Thus, any amounts of the components may be combined together by any means to arrive at the composition of this invention which induces an immune response, preferably a CD8+ T cell or CTL response. In order to function as a composition of this invention, all that is necessary is that an immune response, preferably a CD8+ T cell or CTL response be induced. However, a composition of this invention may also be determined as effective by amelioration or elimination of the condition from which the patient to whom the composition is administered is suffering. Each component in the composition may be bound to one or more of the other components in any way. For example the antigen and the immunomodulator may both be molecularly bound to the carrier or may be encapsulated into the carrier.

The composition of this invention uses a pH-sensitive liposome as a carrier into which is encapsulated an antigen(s) and immunomodulator(s) as defined herein above. Preparation of pH-sensitive liposomes and encapsulation may be accomplished by methods known to a skilled person. However, several new and particularly important modifications of these procedures will maximize the effectiveness and safety of the process and are part of this invention.

One is to avoid the use of chloroform, one of the standard agent used to solubilize the lipids used to prepare the liposomes, as this agent is difficult to remove from the liposomes and is carcinogenic. The present invention preferably does not include the use of chloroform. Other potentially toxic agents should also be avoided in the preparation of the liposomes.

A second modification is the use of a novel combination of lipids to manufacture the pH-sensitive liposomes, which improves the solubility of the lipids and liposomes and permits the lipids to be solubilized without the use of chloroform. This modification consists of using the combination of the lipids dioleoylphosphatidylethanolamine (DOPE) and cholesteryl hemissucinate (CHEMS) to construct pH-sensitive liposomes instead of DOPE and 1,2-dioleoyl-sn-succinyl glycerol (DOSG) which are the lipids used conventionally. In one embodiment of the present invention, DOSG is not used in making the pH sensitive liposomes.

A third modification is that the amount of lipids used to prepare the liposomes should be as high as possible, as this will increase the proportion of vaccine and of immunomodulator(s) which can be encapsulated into the liposomes. In the particular example of this invention described below the amount of lipid used was 8 mg per 0.4 ml does of vaccine. However, high doses such as 40 to 80 mg lipid per 0.4 ml dose of vaccine (or a proportionally higher or lower dose of lipid based on a larger or smaller volume of vaccine), may be even more effective; since as documented below the incorporation of proteins into liposomes increases with increasing amount of lipid as does ability of the resulting liposomal vaccines to stimulate cellular immune responses. The maximum amount of lipid that can be used to construct liposomes is in the range of 80 mg per 0.4 ml does of vaccine (or a proportionally higher or lower dose of lipid based on larger or smaller volume of vaccine). Higher doses of lipids cannot be used because the liposomes occupy all of the available volume.

A fourth modification, is that the encapsulation should be conducted at an appropriate pH to minimize problems of insolubility of the agents used to prepare the composition and of liposome degradation: A high pH favors solubilization, but too high a pH leads to degradation of liposomes and to antigen denaturation. In the particular application of this invention, the optimal pH is about 8.5 to about 9.0.

A fifth modification relates to the treatment of the liposomes containing encapsulated material, which are normally washed or otherwise treated to remove from the preparation any antigen or immunomodulator(s) which is not encapsulated into the liposome. In the modification described in this invention, the final liposome composition is used without washing so that it contains both encapsulated and non-encapsulated vaccine and immunomodulator(s). This simplifies and speeds the manufacturing process, and makes it easier to standardize the dose of vaccine, immunomodulator(s), and liposome lipid administered to patients as none is lost in the washing process.

A sample protocol for combining the components such that the antigen and the immunomodulator are incorporated into the pH-sensitive liposome is provided in the Methods section below. As noted above, the composition of this invention is made by combining an amount of each component effective to provide a CD8+ T cell response to the antigen.

A preferred amount of pH-sensitive liposome for a composition of this invention is from about 0.1 to about 80.0 mg lipid/0.4 ml dose of vaccine. A particularly preferred amount of pH-sensitive liposome is about 8.0 to 80.0 mg of lipid/0.4 ml dose of vaccine.

Preferred immunomodulators are GM-CSF, IL-2, IL-1 and IL-12. A preferred amount of GM-CSF is from about 1.0 to about 300 µg/vaccine dose. A preferred amount of IL-2 is about 10⁴ to about 6,000,000 units, and a particularly preferred amount is about 2,000,000 international units/vaccine dose. Immunomodulators may be covalently bound to polyalkylene glycols (such as PEG) for added stability.

A preferred antigen is a melanoma antigen, which may be present in the form of a vaccine that is made up of a single or multiple antigens. A particularly preferred vaccine is a polyvalent vaccine prepared from antigens shed by melanoma cells in culture and containing multiple melanoma antigens (in particular any one or more of the melanoma antigens MAGE-1, MAGE-3, MART-1/ Melan-A, tyrosinase, gp100, and antigens expressed by fresh melanoma tissue, with approximate molecular weight of about 36. 45, 59, 68, 79. 89, 95, and 110 kilodaltons) and other antigens shed by melanoma cells in tissue culture. A preferred amount of vaccine is from about 1.0 to about 100 µg/dose of composition, and a particularly preferred amount is about 40 µg/dose of composition.

In accordance with the foregoing, a preferred composition of this invention includes a melanoma vaccine and IL-2, IL-12, and/or GM-CSF encapsulated into pH-sensitive liposomes. The components are provided in amounts effective to induce an immune response, preferably a CD8+ T cell or CTL response to the vaccine, which may be determined by known assays (or more efficiently by the assay of this invention described below). A particularly preferred composition includes about 40 ug/dose of vaccine, about 8.0 to 80.0 mg/dose of pH-sensitive liposome, and about 2x10⁸ international units of IL-2 and/or about 250 µg/dose of GM-CSF.

Methods for treating and preventing conditions by the use of compositions of this invention in amounts effective to induce an immune response that treats or prevents the condition are included in this invention. In particular, the use of compositions for treating and preventing melanoma by administering to a patient a composition of this invention that contains one, or several, melanoma antigen(s) are contemplated. Other conditions that may be so treated would include infectious diseases, for example AIDS, and other types of cancer. All that is necessary is to obtain antigens characteristic of the condition, such as antigens shed by cancerous cells. These antigens may be used in the form of the naturally occurring cell or organism: or as solubilized, non-purified or partially purified extracts of these cells or organisms; or can be isolated or synthesized by techniques known to the skilled person; then such antigens are used to form a composition of this invention, as described above. The methods of this invention are directed to the induction of any type of immune response to the target antigen or antigens which are part of the particular composition, for example any humoral or cell-mediated response. Assays for determining the induction of an immune response are well known. In general, a sample of blood from an immunized patient may be assayed for the presence of antibodies to the target antigen. The sample may also be assayed for the presence of cells that recognize the target antigen. Cellular immune responses can also be assayed by delayed type hypersensitivity responses (DTH) to skin tests to the vaccine or antigen. In either case, a positive result means that an immune response has developed. A filter spot assay of this invention is particularly useful in determining whether a cellular immune response has been induced. Further, amelioration or elimination of the condition in question is also a goal of the methods of this invention. However, application of the methods requires only induction of an immune response,

Compositions of this invention may be administered in any amount effective to induce an immune response in a given patient, as discussed above. A skilled person, based on the patient and the type of formulation being used, will readily determine thus appropriate dose levels. As stated, a dose is effective when an immune response against the antigen in the composition is induced, and immune responses may be detected using assays well known in the art. An example of evaluating effective doses is provided below.

Any formulation of a composition of this invention that induces an immune response as described above is part of this invention. Thus, the compositions of this invention may be conventionally formulated into dosage forms appropriate for oral administration (such as pills, lozenges, capsules), administration by inhalation (such as aerosol or mist), suppositories, etc. Thus a composition of this invention may also include conventional inactive ingredients such as excipients, stabilizers, preservatives, dyes, flavorants, etc. A composition of this invention may also include other active ingredients. However, preferred formulations are those developed for injection, which may be for example intradermal, subcutaneous, intramuscular or intravenous.

With regard to an injectable formulation of this invention, a preferred dosage regimen is intradermal injections given every two to three weeks for four doses, then monthly for three months or until a desired immune response is obtained and then every three to six months for a total of two to five years.

The examples that follow are provided to illustrate the invention and are not intended to limit the invention in any way. A skilled person will appreciate that a variety of other embodiments are well within the scope of the present invention.

### EXAMPLES

**METHODS:** The methods described below are provided in sufficient detail such that a skilled practitioner will be able to carry out the necessary techniques and obtain any starting materials required to make a particular application of this invention, i.e. the preparation of a polyvalent vaccine for melanoma encapsulated into pH-sensitive liposomes containing the immunomodulator IL-2 and a filter spot assay to measure CD8+ T cell responses which it stimulates.

### Method of Making Conventional Liposomes:

Conventional, pH-insensitive, control liposomes were prepared as described by Andersen (Cancer Res. 50:1853 - 1856 (1990)) by dissolving 0.50g of dimyristoylphosphatidylcholine (DMPC) (Avanti Polar Lipids, Alabaster, AZ) in 12.5 mol of chloroform : methanol (2:1). This amount of DMPC was sufficient to prepare liposomes for approximately 50 doses of vaccine (4 doses/vial at 40 µg vaccine/dose) at a lipid concentration of 8 mg/dose. The dissolved DMPC was dispensed (1.0 ml each) into sterile 2.0 ml glass vials at 32.0 mg/vial; the solvent was driven off with nitrogen, and the vials were stored at -80°C.

### Method of Making pH-sensitive liposomes:

pH-sensitive liposomes are generated by a substantial modification of a method described by Zhou et al (J Immunol Methods 145:143-152,1991), as follows. The modifications which have been introduced are intended to avoid the use of chloroform to solubilize lipids, as this agent is carcinogenic and is difficult to fully remove from dissolved lipids; to improve the ability to solubilize the lipids and to do so without the use of chloroform; to improve the amount of antigen and immunomodulator protein which is encapsulated into the liposomes; to optimize the pH at which the encapsulation is conducted so as to minimize problems of lipid insolubility and liposome degradation and to simplify the use of the composition by administering it together with non-encapsulated antigen(s) and immunomodulator(s).

Dioleoylphosphatidylethanolamine (DOPE) and cholesteryl hemissucinate (CHEMS) are dissolved at a molar ratio of 6:4 in an amount required to prepare the desired concentration of 8 mg of lipids per dose of the final product. This mixture of lipids can be solubilized to prepare pH-sensitive liposomes using only methanol. By contrast, pH-sensitive liposomes were previously prepared from DOPE and 1,2-dioleoyl-sn-succinyl glycerol (DOSG); a combination of lipids that requires chloroform for effective solubilization. The desired amount of dissolved lipid is transferred to sterile containers, and the solvents driven off under a stream of sterile-filtered nitrogen gas until no trace remain. Lipids prepared in this way are stored at -80 °C until reconstituted. Lipids remain stable at this temperature for over 6 months (Gregoriadis, Liposomes in Biological Systems ed. Gregoriadis & Allison, New York, Wiley, pp.25-96, 1980). Random vials should be tested for aerobic and anaerobic bacteria, fungi, mycoplasma, pyrogens; injected into mice and guinea pigs for general safety tests and other safety tests conducted as mandated by the FDA.

In a specific preparation, stock vials sufficient to prepare one dose of vaccine per vial are prepared as follows: Dioleoylphosphatidylethanolamine (DOPE) and cholesteryl hemissucinate (CHEMS) are mixed in a 6:4 molar ratio by dissolving 487 mg of DOPE and 213 mg of CHEMS in 70 ml of methanol. 0.8 ml of the solution is then transferred to sterile injection vials so that each vial contains the amount of lipid required to prepare a formulation of pH-sensitive liposomes containing 8.0 mg liposome per vaccine dose. The solvent is driven off under a stream of sterile-filtered nitrogen gas until no traces remain. Vials prepared in this way should be stored at -80°C until reconstituted. DOPE and CHEMS are known compounds which may be readily obtained from chemical supply houses, in particular, Amanita Polar Lipids (Alabaster, Alabama).

pH-sensitive liposomes that may be even more effective in stimulating immune responses can be constructed by using higher doses of lipids up to 80 mg lipid per 0.4 ml volume of vaccine; since the amount of protein that can be encapsulated into the liposomes increase with the amount of lipid used to prepare the liposomes and since cellular and CD8 + T cell responses are more common in patients immunized to vaccine encapsulated into liposomes containing higher amounts of lipids. The former relation is illustrated in Table I, and the latter in Table VI. Table I summarizes the results of an experiment in which the amount of radioiodinated GM-CSF that could be encapsulated into pH-sensitive liposomes prepared with different amounts of lipids was measured. The liposomes were prepared exactly as described above, but using different amounts of lipids as indicated in the Table. The amount of lipids studied resulted in pH-sensitive liposomes that contained 8 to 80 mg of lipids per 0.4 ml. The amount of radioiodinated GM-CSF that could be encapsulated into the different formulations of pH-sensitive liposomes was then measured. The encapsulation was performed as described below, using a standard amount of radioiodinated GM-CSF and of a carrier protein (40 mg of human albumin) in all cases. The amount of carrier protein was selected to match that of protein in a standard dose of vaccine. As shown in Table I, only 7.5% of the added GM-CSF was encapsulated into pH-sensitive liposomes prepared with 8 mg of lipid compared to 89% of the same dose of GM-CSF being encapsulated into liposomes prepared from 80 mg of lipids. Higher doses of lipids cannot be used because the liposomes occupy all of the available volume in the solution used to resuspend the lipids and prepare the liposomes.

**TABLE I. EFFECT OF LIPID DOSE ON AMOUNT OF PROTEIN THAT CAN BE ENCAPSULATED INTO LIPOSOMES**

| Amount Lipid Used^{(a)} to Prepare Liposomes | Amount of Protein^{(b)} added to added to liposomes | Amount Protein ^{(b)} encapsulated into liposomes | % encapsulated^{(c)} |
|---|---|---|---|
| 8 mg | 134,200 | 28,300 | 21% |
| 20 mg | 169,400 | 64,800 | 37% |
| 40 mg | 114,400 | 64,600 | 56% |
| 80 mg | 152,300 | 135,400 | 89% |

| | | | |
|---|---|---|---|
| a) mg of protein per 0.4 ml of solution b) cpm of radioiodinated GM-CSF c) % of added radioiodinated GM-CSF remaining in liposomes following 3 washes with normal saline | | | |

### Method of Preparing a Polyvalent Melanoma Antigen Vaccine:

Any antigen or combination of antigens to which it is desired to stimulate an immune response can be used to prepare vaccines encapsulated into pH-sensitive liposomes. In the particular example of this invention the preferred antigen is a polyvalent melanoma antigen vaccine prepared from shed antigens.

The composition of this invention may be designed to elicit an enhanced immune response, in particular a CD8+ T cell response against melanoma cells to treat or prevent melanoma. Individuals skilled in the art will know that such a composition can be prepared from melanoma antigens prepared using a number of different approaches. These include using a single melanoma antigen, or multiple antigens (such as the polyvalent shed antigens vaccine described herein) which are therefore part of a preferred composition. Melanoma antigens may be derived from melanoma cells, which are obtained from the cancerous growths of melanoma patients, and may be used freshly or maintained in culture. The American Type Culture Collection (Gaithersburg, MD) makes available numerous melanoma cell lines. Antigens may specifically be obtained by breaking up cells, or by using materials shed into the cell medium, or can be purified from melanoma cell extracts by biochemical procedures, or synthesized by genetic engineering or other technology. These methods are well known. More than one cell line or antigen may be used, in order to obtain a wider spectrum of melanoma antigens and a more comprehensive CD8+ or CTL response. Assays for determining that an entity is a melanoma antigen are known in the art.

Preferred melanoma antigens may be selected from the known antigens MAGE-1, MAGE-3, MART-1/Melan-A, tyrosinase, MART-I, gp100 and/or from antigens expressed by fresh melanoma tissue, in particular those antigens having approximate molecular weights of about 36, 45, 59, 68, 79, 79, 89, 95, and 110 kilodaltons ('About" used here and elsewhere indicates a range which would be considered reasonable to a skilled practitioner): and/or from antigens shed into the surrounding medium by melanoma cells.

### Melanoma vaccine

In the particular example of this invention described herein, a soluble, partially purified, polyvalent, melanoma antigen vaccine was prepared from the material shed into culture by four lines of melanoma cells adapted to long-term growth in serum-free medium (deposited at ATCC, 12301 Parklawn Drive, Rockville, MD 20852 on April 22, 1993 under numbers SD 1793-1797). Briefly, the cells from SF-SKMel28, SF-M14, SF-M20 and SF-HM54 were incubated at 2x10⁶ cells/ml in serum-free RPMl 1640 for 3 hrs at 37°C. Other melanoma cell lines that express the same melanoma-associated antigens can be substituted. After 3 hrs of incubation, the spent culture medium from each line was collected, centrifuged at 1,000 x g for 15 min to remove particulate material, concentrated by ultra filtration and equal protein amounts of the concentrated shed material collected from the four cell lines pooled. The non-ionic detergent NP-40 was added to a final concentration of 0.5% to the pooled shed material, which was then ultracentrifuged at 100,000 g for 90 min to remove insoluble material, dialyzed against normal saline, and sterilized by filtration through a 0.22 micron filter. The protein concentration of the final product was adjusted to 0.2 mg/ml, and dispensed into pyrogen-free glass vials. For use, the vaccine is admixed with alum or other adjuvants, such as IL-2 liposomes, QS21, or others such as the IL-2 pH-sensitive liposomes described herein. The biochemical and antigenic properties of the vaccine have been published by Bystryn et al (J. Biol. Response Modif., 5:221-224 (1986)) and Reynolds et al (Int. J. Cancer, 72:972-976 (1997)).

**Patients and Immunizations:** Sixty-nine sequentially registered patients (43 men and 26 women, ages between 18 and 75 years) with surgically resected melanoma (American Joint Commission on Cancer (AJCC) stage III) were enrolled in the study conducted at the NYU Medical Center from 1992 to 1995. Other criteria for patient selection were: no evidence of distal metastatic disease, no history of other cancers or other serious systemic disease, positive skin test response to recall antigens or ability to be sensitized to dinitrochlorobenzene (DNCB), no prior therapy for melanoma other than surgery or local radiotherapy. All patients signed informed consent. The patients were immunized with 20 or 40 *µ*g of vaccine protein usually admixed with alum as an adjuvant divided into four equal aliquots, administered intradermally into each extremity every 3 weeks for four times, monthly three times, and at longer intervals thereafter. No other therapy for melanoma or immunosuppressive agents were given while patients were on vaccine treatment. Sera were collected prior to the first immunization, and one week following 6-8 immunizations. The sera were stored at -80°C until used. (See Applebaum et al, J. Natl. Cancer Inst., in press, 1998, for details.)

**Preparation of Antigen Extracts for Immunoblotting:** Two lots of melanoma antigen extracts were prepared, each from equal volumes of fresh, surgically resected, tumor tissue obtained from two separate patients. Lot #1 was prepared from hepatitic metastases resected from one patient, and from splenic metastases from the second patient; lot #2 was prepared from metastases resected from the chest wall and large intestine respectively, of two other patients. To prepare tumor tissue extracts, the melanoma tissue was separated from surrounding normal tissue and necrotic material removed. The extract was prepared so as to be enriched in cell membrane material, which is more likely to contain tumor antigens expressed on the external surface of the cells which are more likely to be biologically relevant. To do this, the tumor tissue was finely diced, homogenized, and nuclear and other cellular debris removed by three cycles of centrifugation at 1,000 g for 10 min. The supernatants were ultracentrifuged at 105,000 g for 1 hr at 4°C, the pellets resuspended in Tris-EDTA buffer (pH 8.0), and ultracentrifuged again at 105,000 g for 20 min at 4°C. The resulting pellets were solubilized in 6mM deoxycholic acid and stored at - 80°C.

A normal autologous tissue extract was prepared similarly from equal volumes of normal uninvolved liver and spleen obtained from the patient used to prepare melanoma antigen extract lot #1. A similar procedure was used to prepare antigen extracts from a control human parotid mixed tumor. Normal tissue from patient #2 was not available. Total protein concentration in all lots of antigens was measured using a Bio-Rad kit (Bio-Rad Laboratories, Paris, France), and normalized to 240 µg/gel.

Assay of Vaccine Induced Melanoma Antibodies: Antibodies to melanoma, and the identity of antigens to which they were directed, were assayed by immunoblotting. Antigen extracts were admixed with Laemmli's buffer (Nature 227:680-85 (1970)) and 2-mercaptoethanol, boiled for 5 min, centrifuged at 6,4000 g for 2 min. and 240 *µ*g of extract/gel was run on 8% SDS-PAGE (Nature 227:680-85 (1970)). Proteins were transferred onto PVDF (Immobilon-P, Millipore, Bedford, MA) membrane in 0.192 M glycine, .025 M tris, pH 8.3 without methanol for 3 h at 30 v, blocked in 5% low-fat milk and washed 3 times in 0.05% Tween 20 in PBS. The membrane was then cut into equal strips containing 10 µg of protein per strip and incubated overnight in 1:50 dilution of patient's serum (unabsorbed). The strips were washed 7 times with 0.05% Tween -20 in PBS, incubated for 4 hr in 1:100 dilution of horseradish peroxidase-conjugated anti-human antibody (Fab' fragment-Sigma Co, St. Louis, MO) in 0.3% Tween 20 in PBS, washed 7 times with 0.05% Tween in PBS and incubated with a substrate containing 0.01% hydrogen peroxide and 0.5 mg/mi 4-chloro-1-naphthol. Vaccine-induced antibodies were evidenced by bands which were present in post-vaccine treatment sera but absent or present in lesser density (50% of greater decrease) in baseline serum obtained from the same patient prior to vaccine treatment. (See Applebaum et al, J. Natl. Cancer Inst., in press, 1998, for details.)

**Identification of melanoma-associated antigens that are immunogenic in humans and expressed *in vivo.*** By using the above procedure, multiple antigens associated with melanoma that are both immunogenic in humans and expressed *in vivo* were identified. These antigens had molecular weights of about 36, 45, 59, 68, 79, 85, 89, 95 and 110 kD.

This was done by investigating the ability of the vaccine treatment to stimulate antibodies to melanoma antigens expressed in vivo. The pattern and level of melanoma antibodies in sera collected from 69 patients with surgically resected AJCC stage III malignant melanoma prior to vaccine treatment and one week following 6-8 immunizations. The same melanoma antigen extract (lot #1), pooled from metastases resected from two patients, was used for all assays. The results are illustrated in Figure 3 and summarized in Table II. Vaccine treatment induced a new antibody response, or augmented a pre-existing response, to one or more antigens expressed in surgically resected melanoma tissue in 35 (51%) of the patients. The antibodies were directed to antigens of molecular weights of about 45, 59, 68, 79, 89, 95 and/or 110 kD. Vaccine-induced immune responses were directed most commonly to the p110 and to the p68 (in 33% and 25% of patients respectively) and less commonly to the p89 and p45 (in 10% and 15% of patients respectively).

The specificity of the antigens defined by vaccine-induced antibodies was investigated by measuring their expression in extracts of surgically resected normal tissue and non-melanoma tumor, using as a probe post-vaccine treatment sera with high levels of antibodies to the target antigens. The sera used to identify each antigen are provided in Table III. The tissues tested included normal autologous tissues obtained from sites (liver in one patients, spleen in the other) adjacent to the metastases used to prepare melanoma antigen extract lot #1; another melanoma antigen extract (lot #2) prepared from metastases resected from two other patients; and an unrelated tumor (a parotid mixed tumor). All extracts were prepared identically and tested at identical protein concentrations. The results are summarized in Table III. None of the melanoma antigens targeted by these antibodies were detected in autologous normal tissue. None, but p68 and p89, were detected in extracts of the parotid tumor. The antigens were commonly expressed in melanoma, as all were detected in a second lot of melanoma antigen extract (lot #2) prepared from two additional patients.

In a subsequent experiment, immunization to the same vaccine was found to stimulate antibody responses to two other antigens of molecular weights 36 kD and 85 kD. The antigen source in this experiment was a membrane extract prepared from fresh melanoma tissue obtained from the patient's own melanoma. These antigens are particularly important for vaccine construction for the reason that they can induce immune responses to a patient's own tumor.

The 36, 45, 59, 68, 79, 85, 89, 95, and 110 kD antigens were identified using as probes vaccine-induced antibodies. The source of these antibodies were individual patients, which are identified by code numbers which are provided in Table III. (The 36 kD and 85 kD antigens were identified using sera code number 338). One skilled in the art will recognize that these antigens can also be identified with monoclonal antibodies raised to the individual antigen.

| TABLE II | | | |
|---|---|---|---|
| Generation of vaccine-induced antibodies which react with antigens present in fresh melanoma tissue | | | |
| No. (%) Patients with Antibody Response to Melanoma Vaccine Immunization (a) (n=69) | | | |
| Melanoma Antigen (kD) | Vaccine-Induced Responses (b) | Vaccine-Enhanced Responses (c) | Any Antibody Response to Immunization |
| 110 | 9 (13%) | 14 (20%) | 23 (33%) |
| 95 | 10 (15%) | 5 (7%) | 15 (22%) |
| 89 | 2 (3%) | 5 (7%) | 7 (10%) |
| 79 | 9 (13%) | 2 (3%) | 11 (16%) |
| 68 | 10 (15%) | 7 (10%) | 17 (25%) |
| 59 | 9 (13%) | 6 (9%) | 15 (22%) |
| 45 | 8 (12%) | 2 (3%) | 10 (15%) |
| Any Antigen | 23 (33%) | 24 (35%) | 35(51%) |
| (a) Measured by immunoblot analysis using a membrane fraction of refresh melanoma tissue as the antigen source | | | |
| (b) Antibody band present in post- but not pre-treatment serum in same patient | | | |
| (c) Density of antibody band greater in post- than pre-treatment serum | | | |

| TABLE III | | | | | |
|---|---|---|---|---|---|
| Tissue distribution of melanoma antigens defined by antibodies induced by vaccine immunization | | | | | |
| Identification Code of Vaccine-induced Antibody | Melanoma Antigen (kD) | Melanoma Antigen Lot #1 (a) | Melanoma Antigen Lot #2 (b) | Normal Autologous Tissue (c) | Unrelated Tumor (d) |
| 162 | 95 | + | +++ | - | NT |
| 68 | 89 | ++ | +++ | - | + |
| 64 | 79 | ++ | ++ | - | - |
| 68 | 68 | +++ | +++ | - | + |
| 108 | 59 | ++ | ++ | - | NT |
| 108 | 51 | ++ | +++ | - | - |
| 108 | 45 | +++ | +++ | - | - |
| 108 | 110 | ++ | +++ | - | |
| (a) Prepared from metastatic nodules obtained from the liver and spleen of two different patients | | | | | |
| (b) Prepared from metastatic nodules obtained from the chest wall and bowel of two different patients | | | | | |
| (c) Normal tissue obtained from autologous liver and spleen from patients utilized for the preparation of melanoma pool # 1 | | | | | |
| (d) Parotid mixed tumor | | | | | |

### Source of interleukin-2 (IL-2)

IL-2 can be obtained from a commercial source such as the Chyron corporation.

### Encapsulating vaccine and immunomodulator into pH-sensitive liposomes

To incorporate antigens such as a vaccine, and immunomodulators such as IL-2, IL-12, IL-1, GM-CSF or other immunomodulators into the liposomes, vials or other containers containing dried lipids are warmed to room temperature, the desired amount of vaccine and IL-2 or other immunomodulators is added to each vial, and the solution is mixed rapidly as with a vortex apparatus. An important aspect of this procedure, which differs from other approaches of making pH sensitive liposomes, is that the pH of the solution is adjusted to 8.5-9.5, since solubilization of lipids will improve at higher pH but liposomes will degrade once the pH is above 10.0. This can be done by using as a diluent for the immunomodulator(s) and vaccine a buffer of the appropriate pH, as indicated in the example that follows.

Multilamellar liposomes containing encapsulated antigen(s) and immunomodulator are then formed by subjecting the preparation to 3 cycles of freeze/thaw/sonication. For each cycle, the container is rapidly frozen in liquid nitrogen, thawed slowly to 27°C followed by 30 sec sonication (Sonic Needle Sonicator, Sonic Needle Corp, Farmingdale, NY). Vaccine can be incorporated into liposomes together with an immunomodulator immediately prior to use to minimize loss of vaccine and immunomodulator due to leakage from liposomes during prolonged storage. In addition, the liposome encapsulated vaccine and immunomodulator can be used without removing from the composition antigen or immunomodulator which has not been encapsulated into the liposomes as this is a simpler and more rapid procedure, and as the total amount of vaccine, immunomodulator(s), and liposome lipid can be controlled more easily and accurately. Alternatively, vaccine and immunomodulator may be pre-packaged and stored for prolonged periods prior to use. This may reduce the amount of vaccine and cytokine present inside as opposed to outside the liposomes. However, the resultant partial loss in activity may be offset by the convenience of use of the pre-packaged product.

In a specific preparation using a polyvalent shed melanoma vaccine and IL-2, vials containing 8 to 80.0 mg of dried lipids are warmed to room temperature. To each vial is added 0.2 ml of vaccine (200 ug/ml) adjusted to pH 8.5-9.0 and 0.2 ml of 10⁷ U/ml human rIL-2 (Proleukin, Chiron Corp., Emeryville, California) in sterile saline adjusted to pH 8.5-9.0. The desired pH can be obtained by diluting the vaccine or immunomodulator with an equal volume of a buffer consisting of 300 mM NaCl and 40 mM HEPES buffer, pH 9.0. The solution is mixed rapidly with a vortex apparatus. Multilamellar liposomes are formed by subjecting the preparation to 3 cycles of freeze/thaw/sonication. For each cycle, the vials are immersed and rapidly frozen in liquid nitrogen, thawed slowly at 27° C, followed by 30 sec bath sonication (70% power setting using a Sonic Needle sonicator, Sonic Needle Corp, Farmingdale, NY). This procedure results in a final preparation of pH-sensitive liposomes that contains 1 dose of vaccine per vial, each dose consisting of 40 µg vaccine, 2 million U of IL-2, and 8.0 to 80.0 mg of lipid per 0.4 ml dose of vaccine.

In a specific preparation using polyvalent melanoma vaccine and GM-CSF, vials containing 8.0 to 80.0 mg of dried lipids are warmed to 27°C, and to each is added 0.2 ml of vaccine (200 ug/ml) and 0.2 ml of one of 3 different concentrations of GM-CSF (Lucien, Immune, Seattle, Washington) (0.0125 mg/ml, 0.125 mg/ml or 1.25 mg/ml). All solutions are adjusted to pH 8.5-9 using the above buffer. The solutions are mixed rapidly with a vortex, and multilamellar liposomes formed by 3 cycles of freeze/thaw/sonication. Each vial of reconstituted vaccine will contain 1 dose of vaccine. Each dose will consist of 40 ug vaccine and either 2.5, 25 or 250 ug of GM-CSF and 8.0 to 80.0 mg of lipid in 0.4 ml of vaccine. GM-CSF may be obtained in 500 mg vials to which 0.5 ml of diluent (preferable bacteriostatic water for injection USP 9% benzyl alcohol) is added to give a concentration of 1 mg/ml.

### Stimulation of immune responses to vaccine antigens:

A vaccine encapsulated into pH-sensitive liposomes together with an immunomodulator(s) can be used to stimulate immune responses, including DTH and antigen-specific CD8+ T cell responses by immunizing humans or animals to the preparation. Immunizations can be performed by administering the composition systemically either intradermally, subcutaneously, intramuscularly, intravenously, intranasally; or by topical application.

In a specific immunization procedure, a polyvalent melanoma antigen vaccine encapsulated into pH-sensitive liposomes together with IL-2 was administered intradermally. The total dose of vaccine was split into equal part, and each part was administered into one of the 4 extremities so as to maximize the number of lymph glands stimulated. In patients who had a regional node dissection, no immunizations was given into that extremity to minimize the chance of an infection and as no regional nodes were present to be stimulated in that extremity. Instead a double dose was given into the contraleteral extremity. Immunizations were given initially every 2 to 3 weeks for 4 cycles. Booster doses were then administered monthly for 2 years; and in other cases were given monthly for 3 cycles, every 3 months for 2 cycles, and then every 6 months for 5 years.

In order to determine the effectiveness of a vaccine composition, assays may be used to determine the ability of the vaccine to stimulate antibody and cellular immune responses.

Antibody responses can be measured by a variety of assays which include immunoblotting, ELISA, immunoprecipitation and SDS-PAGE analysis, complement-mediated cytotoxicity and others. Procedures to conduct these assays are well known to those knowledgeable in the art. An example of measuring antibody responses by immunoblotting is provided in Fig. 3. Cellular responses can also be measured by a variety of assays which include delayed type hypersensitivity assay, proliferation assays, cytolytic assays, and cytokine release assays. CD8+ T cellular responses can be measured by in vitro stimulation assays followed by limiting dilution analysis; or preferably by means of a sensitive and convenient new filter spot assay described below, which is part of this invention.

### Assay of delayed type hypersensitivity (DTH) responses

DTH responses are measured by injecting 10 ug of vaccine intradermally into the volar surface of a forearm. The vaccine preparation used does not contain any adjuvants, liposomes or immunomodulator. The largest diameter of induration is measured 24 hrs. later. There is normally no reaction in non-vaccine treated patients. Induration of 5 mm or greater is considered a positive reaction. Vaccine induced DTH responses are calculated by subtracting baseline induration from that present post-vaccine immunization. Vaccine induced responses of 15 mm or larger are graded as strong and those 25 mm or larger as very strong.

### Assay of vaccine induced CD8+ T cell responses:

In a specific example of the filter spot assay, the effector cells, which are Ficoll-Hypaque isolated mononuclear peripheral blood lymphocytes (the blood cell population which includes CD8+ T cells), which may be fresh, or frozen and thawed, are resuspended in RPMI 1640 culture medium supplemented with 10% fetal bovine serum, and depleted of monocytes by adherence to plastic. Half a million or more lymphocytes are then added to each well of a 96 well microtiter plates, the bottom of each well being covered with a PVDF membrane. Such PVDF membrane filter plates can be obtained commercially (Millipore, Bedford, MA). The PVDF membrane is precoated with monoclonal antibody to human IFN-gamma (Biosource International, Camarillo, CA), a cytokine secreted by activated CD8+ T cells. Monoclonal antibodies to other cytokines secreted by antigen-activated CD8+ T cells can be substituted for IFN-gamma antibody. The plates are preceded with 12.000 to 20,000 (preferably 15,000) target cells/well and, if desired, target peptide at 10-20 nM for the target cells to take up and present.(Dissolving the target peptide in DMSO and diluting with medium provides superior results). The target cells may be conventionally pulsed with 10-20 nM of target peptide at any time prior to addition of effector cells. However, it is preferred to add the peptide just prior to the addition of the effector cells and allow it to remain in the wells for the duration of the assay. Target cells in this example are an HLA-A2.1 positive variant of SFM20, one of the melanoma cell lines used to prepare the vaccine described above. Monoclonal antibody IVAI2 (to human class II antigens HLA-DR DP, DQ) is added to all wells at 30 µg/ml after the addition of the target cells and before the addition of the effector cells to prevent melanoma targets from presenting class II antigens. The phenotype and HLA restriction of responding cells is determined by addition of 30 µg/ml of the antibodies anti-CD4 (OKT4), anti-CD8 (OKT8), anti-CD56, or anti-HLA class I (W6/32) (PharMingen, San Diego, CA) to some of the wells. A preferred method of preparing the blocking antibodies is by purifying them in dialysis tubing to provide concentrated, nondenatured antibodies uncontaminated with purification agents. Assays are preferably performed in duplicate. After 48 h incubation at 37°C and 5% CO₂, the plates are washed with PBS-Tween20, incubated overnight with goat anti-IFN-gamma (R & D, Minneapolis, MN), and then with biotinylated rabbit anti-goat Ig (Sigma, St Louis, Mo) for 2 hr followed by extravidin alkaline phosphatase (Sigma) for one hr. Spots representing individual T cells that have been stimulated by antigen and release IFN-gamma are visualized with BCIP/NBT (KPL, Gaithersburg, MD) and counted on a dissecting microscope. Other procedures of visualizing secreted cytokine can be substituted. Identification of responding cells as CD8+ and the HLA restriction of the responses, may be determined as follows or as described. (Van Der Brugge et al. Eur. J. Immunol. 24:3038,1994; Kawasaki et al. J. Exp. Meth. 180:347,1994; 59; 60). The number of CD8+ cells is calculated as the number of IFN-gamma secreting cells in wells without antibodies minus the number of IFN-gamma secreting cells in wells with anti-CD8 antibodies. Results are expressed as the number of antigen-reactive, lymphokine-secreting cells/500,000 peripheral blood lymphocytes (PBL) (King et al. J. immunol. Meth. 132.37-43, 1990). The number of antigen-specific CD8+ T cells is calculated by subtracting from the number of CD8+ T cells reacting to the test antigen the number of CD8+ T cells reacting to target cells pulsed with a control CD8 peptide epitope that is presented by the same class I HLA molecule as the test peptide but is derived from an unrelated antigen. The assay may be considered positive if there are five or more antigen-specific CD8+ T cells per 500,000 PBL. It is an advantage of this assay that it is sensitive enough to detect less than 5 CD8+ T cells per 500,000 PBL. The number of vaccine induced, antigen-specific CD8+ T cells is calculated by subtracting the number of antigen-specific CD8 T cells present at baseline prior to vaccine treatment from that present following immunization to the vaccine. Details of the procedure can be found in Reynolds et al, Int J Cancer, Jan. 1998.

Specific melanoma peptides used in this example are HLA-A2 restricted CD8 epitope on the melanoma associated antigens MAGE-3 (FLWGPRALV) (SEQ ID NO:1), MART-I (AAGIGILTV) (SEQ ID NO:2); tyrosinase (YMNGTMSQV) (SEQ ID NO:3) and gp-100 (YLKKIKNSL) (SEQ ID NO:4) and as a control on (YLKKIKNSL) an A2-restricted CD8 epitope on CSP, a surface protein of falciparum malaria. These peptides are obtained from Biopolymers Laboratory (Harvard Medical School, Cambridge, Mass). The ability of this assay to detect and quantitate a vaccine-induced CD8+ T cell response to a peptide of the melanoma antigen MAGE-3 is illustrated in Figure 1. Following 4 vaccine immunizations, there is a striking increase in the number of T cells reactive to MAGE-3 peptide pulsed, HLA-A2+, target cells. The T cells are CD8+ as reactivity is abrogated by antibody to CD8 but not by anti-CD4: class I HLA-restricted as reactivity is abrogated by antibody to HLA-class I; specifically directed to MAGE-3 as there is no reactivity to the same targets pulsed with a control HLA A2-restricted peptide recognized by CD8 + cells on a control antigen (the malaria protein CSP); and are vaccine-induced as reactivity is absent prior to vaccine treatment.

The assay is reproducible. Similar results were obtained when the same specimen of blood lymphocytes was tested on two occasions in 3 patients (see Table IV).

**TABLE IV REPRODUCIBILITY OF FILTER SPOT ASSAY FOR PEPTIDE-SPECIFIC CD8+ T CELLS IN PERIPHERAL BLOOD**

| PATIENT | NUMBER OF PEPTIDE-SPECIFIC CD8+ T CELLS¹ | |
|---|---|---|
| | Assay 1 | Assay 2 |
| #405² | 36.0± 1.3 | 35.3±2.10 |
| #420³ | 19.3±0.80 | 20.4±1.10 |
| #428³ | 11.6±0.00 | 13.6±0.53 |

| | | |
|---|---|---|
| ¹ number of CD8+ T cells/500.000 peripheral blood lymphocytes (= S.E.) post 4th immunization ² CD8+ T cells reacting to Melan A/MART-1 (27-35) ³ CD8+ T cells reacting to MAGE-3 (271-278) | | |

### Preparation of Target Cells for the Filter Spot Assay of CD8+ T Cell Response

With regard to the target cells used in the filter spot assay described above, any cell that carries a target antigen may be used, not necessarily an antigen presenting cell. However an antigen presenting cell which presents a selected target antigen and which in addition expresses a specific type of class I HLA allele is preferred, and may be obtained by isolation or by genetic engineering, for example as described immediately below. There are different types, i.e. alleles, of class I HLA molecules. A2 is the allele which was used for this example because it is the one which is most frequently expressed by individuals at high risk of developing melanoma. To measure CD8+ T cells in individuals of different class I HLA phenotype, one must use target cells that express those allele. The SFM2O target cell line may be used in the assay because it expresses a melanoma antigen from the vaccine described above. SFM20 is A2 negative, but the variant used in this example has been transfected by known methods with the human-HLA A2.1 gene or as control with the murine Db gene. The cells used as target cells in the CD8 T cell response assay above were prepared as follows: A 5~ 1 kb HLA-A2.1 genomic fragment obtained by Hindill digestion of PuC plasmid is cloned into the Hindill site of a ph~-Apr-neo vector. The resulting vector is linearized with Pvul and transfected into SFM20 cells using a lipofectin reagent (Gibco, Gaithersburg, MD) according to manufacturer's instructions. G-41 8 resistant colonies are selected in the presence of 1 mg/ml G-418 and screened for HLA-A2 expression by indirect immunofluorescence staining with BB7.2 antibodies. Pretreating these target cells with IFN-gamma for two days prior to use to maximize HLA molecule expression provides superior results.

Target cells are harvested at log phase, checked for level of HLA expression by FACS analysis, irradiated with 4,000 rads and frozen in 10% DMSO in fetal bovine serum at 106 cells/vial in liquid nitrogen (this treatment provides highly consistent results since the same target cell preparation may be used).

### EXAMPLES

### Example 1: Use of pH-sensitive liposome compositions

### a. Use in mice.

A study in mice was performed using a polyvalent murine B16 melanoma vaccine encapsulated together with IL-2 (Gershman et al, Vaccine Res 3:83-92, 1994) into pH-sensitive liposomes. The melanoma vaccine used for these studies was prepared from sonicated cells, rather than from shed material that is used to formulate the human vaccine.

Groups of 10 C57B1/6 mice were immunized to the vaccine encapsulated into various dose levels of pH-sensitive liposomes (0.2 to 18.3 mg total lipid/immunizations) together with or without human IL-2. Immunizations were given s.c. weekly x 4. As controls, similar groups of mice were immunized to pH-sensitive liposomes containing IL-2 but no vaccine or to saline alone. There was no systemic toxicity as evaluated by loss of weight or death. Local toxicity was minimal, consisting of small local granulomas which did not ulcerate in approximately 40% of mice. Vaccine-induced tumor protective immunity was highest in mice immunized to vaccine encapsulated into pH-sensitive liposomes together with IL-2 (see Figure 1 and Table V). This was a result of the combination of IL-2 and pH-sensitive liposomes, as vaccine-induced protective immunity in this group was higher than in mice immunized to vaccine encapsulated into pH-sensitive liposomes lacking IL-2. Cellular response (measured by DTH responses to skin tests to melanoma cells) was also highest in the group that received vaccine + IL-2 encapsulated into pH-sensitive liposomes. The pH-sensitive liposomes potentiated tumor protective immunity at all doses studied.

**TABLE V**

| **EFFECT OF pH-SENSITIVE LIPOSOMES ON THE IMMUNOGENICITY OF A MURINE B16 MELANOMA VACCINE** | | | | | |
|---|---|---|---|---|---|
| MELANOMA VACCINE (a) | LIPOSOMES^{e} | IL-2 | ANTIBODY RESPONSE (b) | CELLULAR RESPONSE (c) | SURVIVAL @ 10 weeks(d) |
| 150µg | pH-sensitive | 10⁴U | 41.3=11.2% | 0.089±0.021 | 80% |
| 150µG | conventional | 10⁴U | 35.9± 7.2% | 0.063±0.020 | 50% |
| 0 | pH sensitive | 10⁴U | 7.1±5.8% | 0.054±0.030 | 30% |
| 0 | none | none | 0±6.0% | 0.062±0.019 | 20% |

| | | | | | |
|---|---|---|---|---|---|
| a) 10 mice/group immunized weekly x 4 b) measured by complement-mediated cytotoxicity 2 weeks post 4 immunizations c) measured by DTH response to melanoma cells 2 weeks post 4th immunization d) % mice alive 10 weeks after challenge with a lethal number of murine B16 melanoma cells e) 3.66 mg per vaccine dose | | | | | |

### b. Use in Humans

The following clinical trial was conducted to examine the ability of a melanoma vaccine encapsulated into pH-sensitive liposomes to stimulate cellular (DTH) and CD8 + T cell responses, to examine the effect of the dose of lipid used to prepare the liposomes on the ability of the vaccine to induce these responses, and to compare the effectiveness of pH-sensitive as opposed to conventional (non-pH sensitive liposomes) in inducing these responses.

Seventeen patients with surgically resected. American Joint Commission on Cancer stage IIB or III malignant melanoma were randomly assigned to treatment with a standard does of the polyvalent shed melanoma vaccine described above which was encapsulated together with a standard dose of IL-2 (10.000 International Units/vaccine dose) into one of 3 different formulations of liposomes. Two of the formulations were of pH-sensitive liposomes containing either 0.4 mg or 8 mg of lipids per vaccine dose, the third formulation was conventional liposomes (not pH-sensitive) containing 8 mg of lipids per dose. All patients were treated with the same dose of vaccine and IL-2. The dose of IL-2 used was less than optimal, a more effective dose being in the range of 2 million units/vaccine dose.

The ability of the different types of liposomes to stimulate cellular immune responses was compared by measuring DTH skin test responses to the vaccine and CD8 + T cell responses to melanoma associated peptides prior to vaccine treatment and following 4 immunizations. The results are summarized in Table VI.

**TABLE VI. EFFECT OF DIFFERENT TYPES OF LIPOSOMES ON ABILITY OF A POLYVALENT MELANOMA VACCINE TO STIMULATE CELLULAR IMMUNE RESPONSES**

| Liposome Formulation | Dose^{(a)} Lipid | # PTS | % of PTS with DTH^{(c)} | Vaccine Induced CD8 + T Cell Response^{(d)} |
|---|---|---|---|---|
| conventional | 8.0 mg | 6 | 17% | in 0 of 1 pt |
| pH-sensitive | .04 mg | 5 | 20% | in 0 to 1 pts |
| pH-sensitive | 8.0 mg | 6 | 66% | in 2 of 3 pts |

| | | | | |
|---|---|---|---|---|
| a) does of lipid used to prepare liposome/per vaccine dose. b) following 4 vaccine immunizations. c) vaccine induced DTH response 25 mm or greater than baseline measurement in same patient. d) Number of HLA-A2 positive patients with vaccine induced, peptide-specific. CD8 + T cell response directed to A2 restricted peptides on the melanoma-associated antigens MART-1 or tyrosinase. | | | | |

Vaccine treatment stimulated very strong DTH responses (25 mm or greater increase in induration over baseline measurement in the same patient) in 4 (66%) of 6 pts immunized to vaccine + IL-2 in liposomes prepared from a high dose of lipid (8 mg lipid/dose vaccine) compared to in only 20% of patients immunized similarly to the same dose of vaccine and IL-2 encapsulated into pH-sensitive liposomes prepared from a low dose of lipid (0.4 mg lipid/vaccine dose). In addition, strong DTH responses were much more common in patients immunized to vaccine in pH-sensitive liposomes than in those immunized identically to vaccine encapsulated into conventional liposomes prepared from an identical does of (8 mg lipid/vaccine does) lipid.

The number of CD8+ T cells in peripheral blood that reacted specifically to HLA-A2 restricted, CD8 peptides on the melanoma antigens tyrosinase and MART-1 were measured before vaccine treatment and following four immunizations in the five patients in this study who were HLA-A2 positive and eligible for this analysis. CD8+ T cells specific to these antigens were rarely present prior to vaccine treatment. Following vaccine treatment the level of CD8+ T cells to one or the other of these two antigens was augmented in 2 of the 3 HLA-A2 positive patients immunized to vaccine in pH-sensitive liposomes prepared with a high dose of lipids. By contrast, no CD8+ T cells response to these antigens was induced in the one HLA-A2 positive melanoma patient treated similarly to vaccine encapsulated into conventional liposomes or in the other HLA-A2 positive patient immunized to vaccine in pH-sensitive liposomes prepared from a low dose of lipids. These results indicate that encapsulating vaccine + IL-2 into pH sensitive liposomes can result in the stimulation of cellular (DTH and CD8 + T cell) responses to the vaccine, that pH-sensitive liposomes stimulate these responses more effectively than conventional liposomes, and that the pH-sensitive liposomes are more effective when prepared from higher doses of lipids. The method used to prepare the conventional liposomes has been published (Gershmann et al).

## Claims

1. A vaccine composition which comprises:
at least one cancer antigen prepared from shed antigens of a cancer cell;
at least one immunomodulator selected from IL-1, IL-2, IL-4, IL-6, IL-12 and GM-CSF; and
a carrier which is a chloroform-free pH sensitive liposome comprising dioleoylphosphatidylethanolamine (DOPE) and cholesteryl hemisuccinate (CHEMS) which are formed at a pH of 8.5-9.5, to encapsulate said cancer antigen and said immunomodulator and to deliver them together to immune cells when administered to a human, such that said antigen induces a CD8+ T cell response.

2. A composition according to claim 1 in which the liposomes are formed at the highest concentration of lipid possible, to maximise the amount of antigens and immunomodulators that can be encapsulated.

3. A composition according to claim 1 or 2 wherein the mixture of antigen(s), immunomodulator(s) and carrier are used without removing from the mixture the antigen(s) and immunomodulator(s) which have not been bound or incorporated into the carrier.

4. The vaccine composition according to any one of claims 1 to 3 for use in a method of treatment of the human or animal body.

5. The use of a vaccine composition according to any one of claims 1 to 3 in the manufacture of a medicament to induce an immune response against a cancer and so treat or prevent the cancer.

6. The use according to claim 5 wherein the medicament is to induce an immune response to melanoma and so to treat or prevent melanoma.

## Patentansprüche

1. Vakzinenzusammensetzung, umfassend:
zumindest ein Krebsantigen, das aus von einer Krebszelle abgespaltenen Antigenen hergestellt ist;
zumindest einen aus IL-1, IL-2, IL-4, IL-6, IL-12 und GM-CSF ausgewählten Immunmodulator; und
einen Träger, bei dem es sich um ein chloroformfreies, pH-empfindliches Liposom handelt, das Dioleoylphosphatidylethanolamin (DOPE) und Cholesterylhemisuccinat (CHEMS) umfasst, die bei einem pH von 8,5-9,5 gebildet werden, um das Krebsantigen und den Immunmodulator zu verkapseln und sie bei Verabreichung an einen Menschen zusammen zu Immunzellen zuzuführen, sodass das Antigen eine CD8+-T-Zell-Reaktion induziert.

2. Zusammensetzung nach Anspruch 1, worin die Liposomen mit der höchstmöglichen Lipidkonzentration gebildet werden, um die Menge an Antigenen und Immunmodulatoren, die verkapselt werden kann, zu maximieren.

3. Zusammensetzung nach Anspruch 1 oder 2, worin das Gemisch aus Antigen(en), Immunmodulator(en) und Träger verwendet wird, ohne dass das/die Antigen(e) und der/die Immunmodulator(en) entfernt werden, die nicht gebunden oder in den Träger inkorporiert wurden.

4. Vakzinenzusammensetzung nach einem der Ansprüche 1 bis 3 zur Verwendung in einem Verfahren zur Behandlung eines menschlichen oder tierischen Körpers.

5. Verwendung einer Vakzinenzusammensetzung nach einem der Ansprüche 1 bis 3 bei der Herstellung eines Medikaments zur Induktion einer Immunantwort gegen einen Krebs und so zur Behandlung oder Vorbeugung des Krebses.

6. Verwendung nach Anspruch 5, worin das Medikament zur Induktion einer Immunantwort auf ein Melanom und so zur Behandlung oder Vorbeugung des Melanoms dient.

## Revendications

1. Composition de vaccin qui comprend:
au moins un antigène du cancer préparé à partir d'antigènes répandus d'une cellule cancéreuse;
au moins un immunomodulateur sélectionné parmi IL-1, IL-2, IL-4, IL-6, IL-12 et GM-CSF; et
un support qui est un liposome sensible au pH sans chloroforme comprenant de la dioléoylphosphatidyléthanolamine (DOPE) et de l'hémisuccinate de cholestéryle (CHEMS) qui sont formés à un pH de 8,5-9,5 pour encapsuler ledit antigène du cancer et ledit immunomodulateur et pour les délivrer ensemble à des cellules immunes lors d'une administration à un humain de façon que ledit antigène induise une réponse des cellules T CD8+.

2. Composition selon la revendication 1 dans laquelle les liposomes sont formés à la plus forte concentration en lipides possible pour maximiser la quantité des antigènes et des immunomodulateurs qui peuvent être encapsulés.

3. Composition selon la revendication 1 ou 2 où le mélange du ou des antigène(s), du ou des immunomodulateur(s) et du support sont utilisés sans éliminer du mélange le ou les antigène(s) et le ou les immunodulateur(s) qui n'ont pas été liés ou incorporés dans le support.

4. Composition de vaccin selon l'une quelconque des revendications 1 à 3 utilisée dans une méthode de traitement du corps humain ou animal.

5. Utilisation d'une composition de vaccin selon l'une quelconque des revendications 1 à 3 dans la fabrication d'un médicament pour induire une réponse immune contre un cancer et ainsi traiter ou prévenir le cancer.

6. Utilisation selon la revendication 5 où le médicament est pour induire une réponse immune à un mélanome et pour traiter ainsi ou prévenir le mélanome.


### SEQUENCE LISTING

(1) GENERAL INFORMATION:
   (i) APPLICANT: BYSTRYN. JEAN-CLAUDE
   (ii) TITLE OF INVENTION: pH-SENSITIVE LIPOSOMES AND OTHER TYPES OF ENCAPSULATED VACCINES CONTAINING IMMUNOMODULATORS, AND METHODS FOR MAKING AND USING
   (iii) NUMBER OF SEQUENCES: 4
   (iv) CORRESPONDENCE ADDRESS:
      (A) ADDRESSEE: NIXON & VANDERHYE P.C.
      (B) STREET: 1100 NORTH GLEBE ROAD. 8TH FLOOR
      (C) CITY: ARLINGTON
      (D) STATE: VA
      (E) COUNTRY: USA
      (F) ZIP: 22201
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.30
   (vi) CURRENT APPLICATION DATA:
      (A) APPLICATION NUMBER: US
      (B) FILING DATE:
      (C) CLASSIFICATION:
   (viii) ATTORNEY/AGENT INFORMATION:
      (A) NAME: SADOFF. 3.J.
      (B) REGISTRATION NUMBER: 36.663
      (C) REFERENCE/DOCKET NUMBER: 2494-4
   (ix) TELECOMMUNICATION INFORMATION:
      (A) TELEPHONE: 703-816-4000
      (B) TELEFAX: 703-816-4100
(2) INFORMATION FOR SEQ ID NO:1:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:1:
   Phe Leu Trp Gly Pro Arg Ala Leu Val
   1               5
(2) INFORMATION FOR SEQ ID NO:2:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:2:
   Ala Ala Gly Ile Gly Ile Leu Thr Val
   1               5
(2) INFORMATION FOR SEQ ID NO:3:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:3:
   Tyr Met Asn Gly Thr Met Ser Gln Val
   1               5
(2) INFORMATION FOR SEQ ID NO:4:
   (i) SEQUENCE CHARACTERISTICS:
      (A) LENGTH: 9 amino acids
      (B) TYPE: amino acid
      (C) STRANDEDNESS: single
      (D) TOPOLOGY: linear
   (ii) MOLECULE TYPE: peptide
   (xi) SEQUENCE DESCRIPTION: SEQ ID NO:4:
   Tyr Leu Lys Lys Ile Lys Asn Ser Leu
   1               5